# FASCICULE DE BREVET EUROPEEN

(11) **EP 0 965 588 B1**
(45) Date de publication et mention de la délivrance du brevet: **10.03.2004**
(21) Numéro de dépôt: 99401497.5
(22) Date de dépôt: 17.06.1999
(51) Int. Cl.: C07D 211/42, C07D 211/70, C07D 493/04

(54) **Procédé de préparation de dérivés de la 4-phényl-1,2,3,6-tétrahydropyridine et les produits intermédiaires mis en oeuvre**
Verfahren zur Herstellung von 4-phenyl-1,2,3,6-tetrahydropyridin-derivaten und die dafür verwendeten Zwischenprodukte
Process for the preparation of 4-phenyl-1,2,3,6-tetrahydropyridine derivatives and intermediates used in this process

(30) Priorité: 18.06.1998 FR 9807677
(43) Date de publication de la demande: 22.12.1999
(73) Titulaire: Aventis Pharma S.A., 92160 Antony (FR)
(72) Inventeur: Brion, Francis, 31130 Balma (FR); Richard, Christian, 93110 Rosny Sous Bois (FR)

(56) Documents cités:
- WO-A-97/42949
- FR-A- 2 338 043
- US-A- 4 132 710
- NAIK, RAMACHANDRA G. ET AL: "An antiinflammatory and immunomodulatory piperidinylbenzopyranone from Dysoxylum binectariferum: isolation, structure and total synthesis" TETRAHEDRON (1988), 44(7), 2081-6,1988, XP002093909
- NAGAI, YASUTAKA ET AL: "Studies on psychotropic agents. VI. Synthesis of 1-methylspiro[6- fluoroindan-1,3'-pyrrolidin]-3-one and related compounds" CHEM. PHARM. BULL. (1980), 28(5), 1387-93, 1980, XP002093910
- LYLE, ROBERT E. ET AL: "Nucleophilic opening of the oxirane ring of 1-methyl-4-phenyl-3,4- epoxypiperidine" J. ORG. CHEM. (1967), 32(9), 2873-5,1967, XP002093911

## Description

La présente invention concerne un nouveau procédé de préparation de dérivés de la 4-phényl-1,2,3,6-tétrahydropyridine et les produits intermédiaires mis en oeuvre.

L'article Naik, Ramachandra G. et al. Tetrahedron (1988), 44(7), 2081-6, 1988 décrit des pipéridinylbenzopyranones et leur synthèse. Le brevet US 4,132,710 décrit des dérivés de benzopyrano[3,4-c]pyridine.

L'article Lyle, Robert E. et al. J. Org. Chem. (1967), 32(9), 2873-5, 1967 décrit l'ouverture de cycle oxirane de 1-méthyl-4-phényl 3,4-époxypipérinidine.

L'invention a pour objet un procédé de préparation des composés de formule (I) de stéréochimie cis : 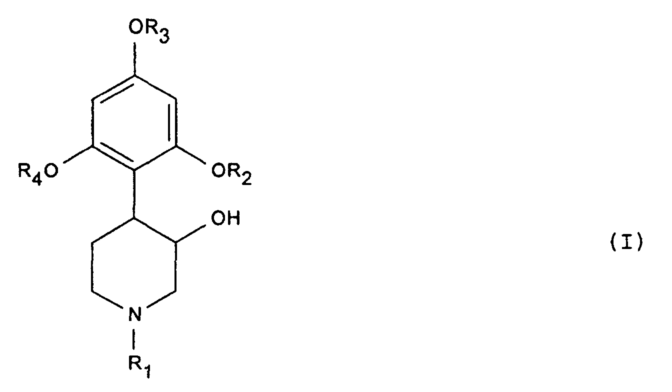 dans laquelle R₁ représente un atome d'hydrogène ou un radical alkyle renfermant jusqu'à 8 atomes de carbone, R₂ et R₄ représentent un radical alkyle renfermant jusqu'à 4 atomes de carbone et R₃ représente un radical alkyle renfermant jusqu'à 8 atomes de carbone, caractérisé en ce que l'on soumet un composé de formule (II) : 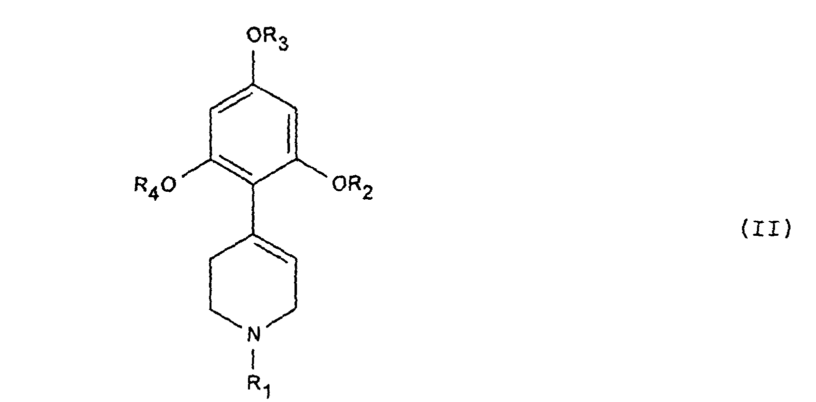 dans laquelle R₁, R₂, R₃ et R₄ conservent leur signification précédente ou un sel d'amine de ce composé, à l'action d'un agent d'halogénation électrophile pour obtenir le composé de formule (III) : 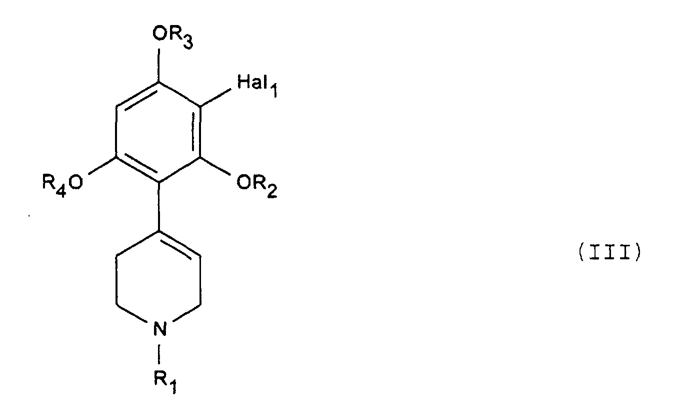 dans laquelle Hal₁ représente un atome d'halogène que l'on soumet à l'action d'un agent d'halohydroxylation pour obtenir le composé de formule (IV) : 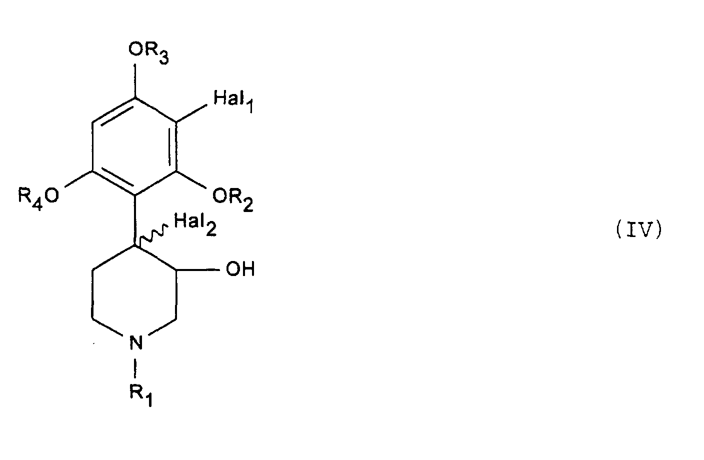 dans laquelle Hal₂ représente un atome d'halogène, que l'on soumet à l'action d'une base, pour obtenir le composé de formule (V) : 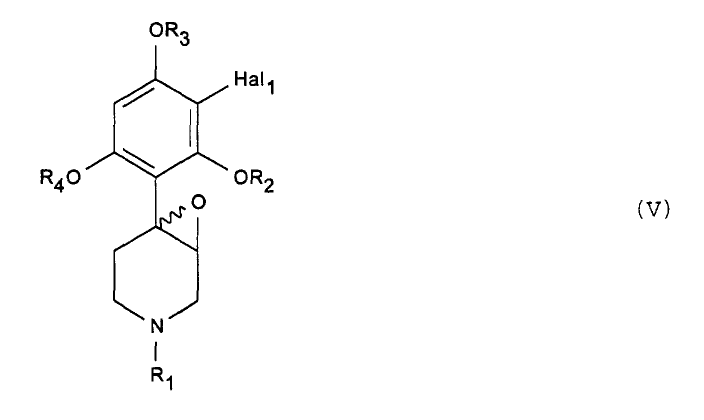 que l'on soumet à l'action d'un agent de réduction diastéréo-sélectif du groupe époxy pour obtenir le composé racémique de formule (VI) ayant la stéréochimie cis : 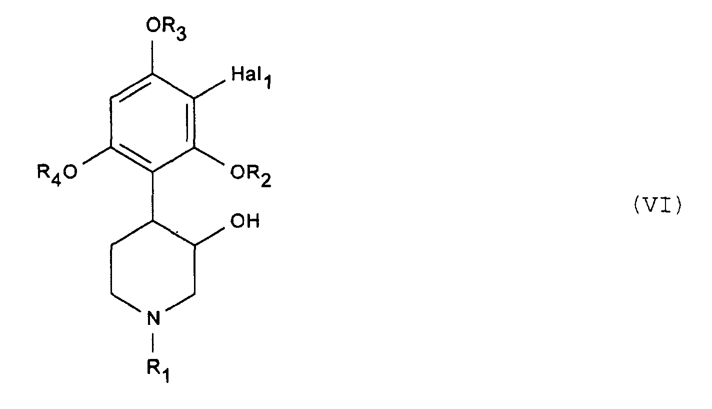 que l'on soumet à l'action d'un agent de réduction pour obtenir le composé racémique cis de formule (I) : 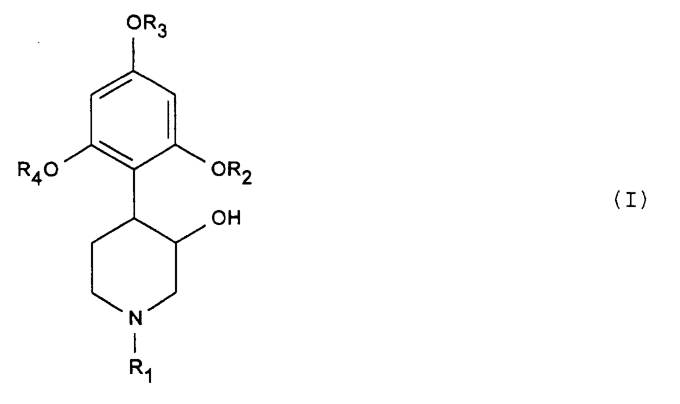 que l'on soumet si désiré, à l'action d'un agent de dédoublement pour obtenir le composé de formule (I) correspondant sous la forme optique voulue.

Les produits de formule (I) sont des produits connus utiles notamment pour la synthèse de produits thérapeutiquement actifs. Les produits de formule (I), leur préparation et leur application dans la préparation de produits actifs sont décrits dans le brevet européen 0241003. Les produits de formule (II) utilisés comme produits de départ du procédé de l'invention sont également des produits connus, décrits dans le brevet européen 0241003.

L'invention a notamment pour objet le procédé caractérisé en ce que R₁ représente un radical alkyle renfermant jusqu'à 4 atomes de carbone ou encore le procédé caractérisé en ce que R₂, R₃ et R₄ représentent un radical alkyle renfermant jusqu'à 4 atomes de carbone et plus particulièrement un procédé caractérisé en ce que R₁, R₂, R₃ et R₄ représentent un radical méthyle.

L'invention a tout spécialement pour objet un procédé caractérisé en ce que le produit de départ est un produit de formule (II) utilisé sous forme de sel d'amine par exemple le sel de l'acide trifluoroacétique ou encore l'acide bromhydrique.

L'invention a notamment pour objet un procédé caractérisé en ce que Hal₁ et Hal₂ sont des atomes de brome.

L'invention a plus spécialement pour objet un procédé caractérisé en ce que l'agent d'halohydroxylation est le N-bromo succinimide.

On peut utiliser également d'autres agents d'halogénation électrophiles comme agents d'halogénation ou d'halohydroxylation, et notamment le N-bromoacétamide, ou la N,N-dibromodiméthylhydantoïne.

L'invention a plus spécialement pour objet un procédé caractérisé en ce que la base utilisée pour créer l'époxyde est la soude ou la potasse.

L'invention a notamment pour objet un procédé caractérisé en ce que les composés de formule (III) et (IV) ne sont pas isolés.

La partie expérimentale exposée ci-après, décrit un procédé où les 2 produits intermédiaires ne sont pas isolés.

L'invention a plus spécialement pour objet un procédé caractérisé en ce que l'agent de réduction diastéréosélective de l'époxy est l'alane AlH₃.

L'invention a également pour objet un procédé caractérisé en ce que l'agent de réduction du composé (VI) est le zinc en milieu alcoolique ou l'hydrure de triN-butylétain.

L'invention a notamment pour objet un procédé où les produits préparés (V), (VI) et (I) sont dédoublés, par exemple un procédé caractérisé en ce que l'agent de dédoublement est un acide de formule : 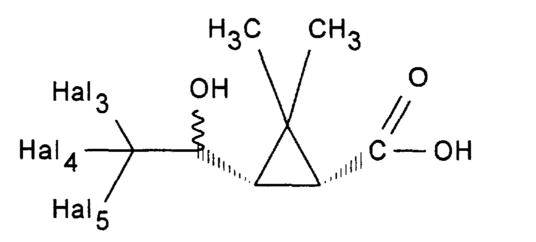 dans laquelle Hal₃ représente un atome de chlore, de brome ou un groupement CF₃ et Hal₄ et Hal₅ représentent un atome de chlore ou de brome, par exemple un procédé où le dédoublement est réalisé au moyen de l'un des 3 acides suivants :

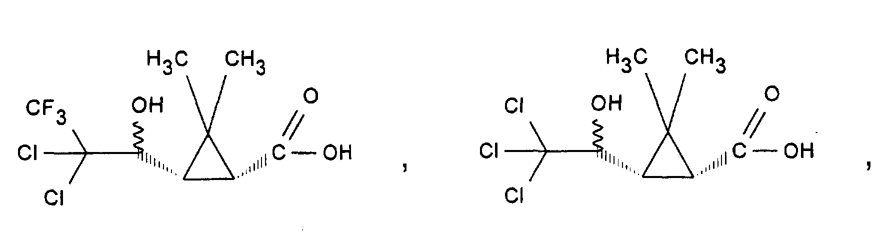

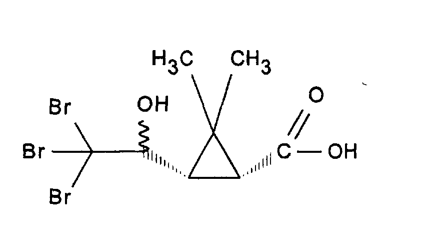

L'invention a plus spécialement pour objet un procédé où l'agent de dédoublement est le :
[1R-(1R*,3S*)] acide 3-(2,2-dichloro-1-hydroxy-3,3,3-trifluoropropyl)-2,2-diméthyl-cyclopropanecarboxylique.

Les produits mis en oeuvre au cours du procédé de l'invention sont des produits nouveaux et sont en eux-mêmes un objet de la présente invention.

L'invention a plus particulièrement pour objet à titre de produits chimiques nouveaux, les produits de formules (III), (IV), (V) et (VI) mis en oeuvre dans la partie expérimentale.

L'invention a plus spécialement pour objet à titre de produits chimiques nouveaux, les produits de formule (V) et tout spécialement le produit de formule (V) décrit ci-après.

Les exemples suivants illustrent l'invention sans toutefois la limiter.

### EXEMPLE : [3S-cis(-)]-1-méthyl-4-(2,4,6-triméthoxyphényl)-3-pipéridinol

### A) Préparation du 6-(3-bromo-2,4,6-triméthoxyphényl)-3-méthyl-7-oxa-3-aza-bicyclo(4,1,0)heptane.

### Stade de salification :

On refroidit à 15°C, une suspension renfermant 100 g de 1-méthyl-1,2,3,6-tétrahydro-4-(2,4,6-triméthoxyphényl)-pyridine et 533 ml de diméthylsulfoxyde. On ajoute en 2 minutes 133 ml d'eau déminéralisée à 20°C. On refroidit à 5°±1°C et ajoute 32 ml d'acide trifluoroacétique. On agite 15 minutes à 5°±1°C.

### Stade de bromation, hydrobromation :

### 4-(3-bromo-2,4,6-triméthoxyphényl)-1-méthyl-1,2,3,6-tétra-hydro-pyridine 4-bromo-4-(3-bromo-2,4,6-triméthoxyphényl)-1-méthyl-3-pipéridinol

On ajoute à 5°±1°C, 169 g de N-bromosuccinimide au produit obtenu précédemment. On agite pendant 1 h 30 à 5°±1°C et ajoute 5 g de métabisulfite de sodium.

### Stade d'époxydation :

### 6-(3-bromo-2,4,6-trimétoxyphényl)-3-méthyl-7-oxa-3-aza-bicyclo(4,1,0)heptane

On ajoute en 10 minutes environ, à la solution précédente, 50 ml de lessive de soude à 30 %. On agite à 5°±1°C pendant 15 minutes et ajoute 150 ml de lessive de soude à 30 %. On agite pendant 1 h 30.

### Isolement :

On verse la suspension obtenue ci-dessus, dans 1000 ml d'eau déminéralisée. On agite pendant 1 heure à 7∼10°C, essore et clairce avec de l'eau déminéralisée. On sèche. On obtient 130,6 g de produit recherché.

### B) Préparation du cis(+-)-1-méthyl-4-(2,4,6-triméthoxyphényl)-3-pipéridinol

### Préparation du réactif AlH₃/THF :

On introduit vers 20°C, 6 g d'hydrure de lithium aluminium dans 100 ml de THF. On agite la suspension obtenue pendant 4 heures à 20/25°C. On laisse au repos pendant 20 heures. On agite à 0°±1°C et ajoute 5,3 g de chlorure d'aluminium anhydre. On laisse la température remonter à 20°C et agite 15 minutes à 20°C. On obtient ainsi le réactif de réduction recherché.

### Réaction :

On refroidit la suspension obtenue ci-dessus (AlH₃/THF) à 0°±1°C. On ajoute à 0°±1°C une solution de 20 g de produit préparé au stade précédent et 120 ml de THF. On agite 2 heures à 0°±1°C. On ajoute 100 ml d'une solution normale de soude. On agite pendant 1 heure et laisse la température remonter vers 20°C. On filtre, on clairce plusieurs fois avec 20 ml de THF. On joint le filtrat et les clairçages. On concentre par distillation sous pression réduite pendant 1 heure. On ajoute 300 ml de méthanol à la solution de cis(+-)-4-(3-bromo-2,4,6-triméthoxyphényl)-1-métyl-3-pipéridinol. On agite et ajoute 70 ml de lessive de potasse à 50 %. On introduit 20 g de poudre de zinc. On chauffe au reflux pendant 16 heures. On refroidit à 20°C. On filtre et clairce avec 20 ml de méthanol anhydre. On concentre par distillation sous pression réduite le filtrat et les clairçages. On ajoute à 20°C, 200 ml d'eau déminéralisée. On refroidit à 0°C. On agite 1 heure à 0°C. On essore et clairce avec 20 ml d'eau. On sèche et obtient 13,71 g de produit recherché.

### C) Préparation de [3S-cis(-)]-1-méthyl-4-(2,4,6-triméthoxyphényl)-3-pipéridinol

On chauffe à 70°C, une suspension renfermant 12 g de produit préparé au stade précédent, 12,59 g de [1R-(1R*,3S*)] acide 3-(2,2-dichloro-1-hydroxy-3,3,3-trifluoropropyl)-2,2-diméthyl-cyclopropanecarboxylique et 60 ml d'éthanol. On ajoute à 67°C, 0,12 g d'amorces cristallines sous forme cis (-). On refroidit à 25°C en 5 heures, le mélange réactionnel. On agite 16 heures à 25°C, on essore et rince avec 12 ml d'éthanol. On sèche sous pression réduite à 50°C pendant 16 heures. On obtient 9,97 g de produit recherché brut que l'on recristallise dans la méthylisobutylcétone. On obtient ainsi 7,75 g de produit recherché, salifié auxquels on ajoute 23 ml d'eau déminéralisée et 39 ml d'éther isopropylique. On agite à 20°C, la suspension obtenue, et ajoute 20,1 ml d'une solution d'acide chlorhydrique 2N. On décante et lave la phase aqueuse avec 23 ml d'éther isopropylique. On extrait avec une solution d'acide chlorhydrique 2N. On réunit les phases acides et ajoute 9,7 g de chlorure de sodium. On agite, refroidit la solution obtenue à 0°C et ajoute 7,75 ml d'une lessive de soude à 30°C. On agite entre 0 et -5°C pendant 1 heure 30 minutes. On essore et rince à l'eau déminéralisée. On sèche en étuve à 50°C pendant 16 heures. On obtient 3,65 g de produit recherché.
F = 115°C.
α_{D} = -53°3, C = 2 % CH₃OH.

## Revendications

1. Procédé de préparation des composés cis de formule (I) : 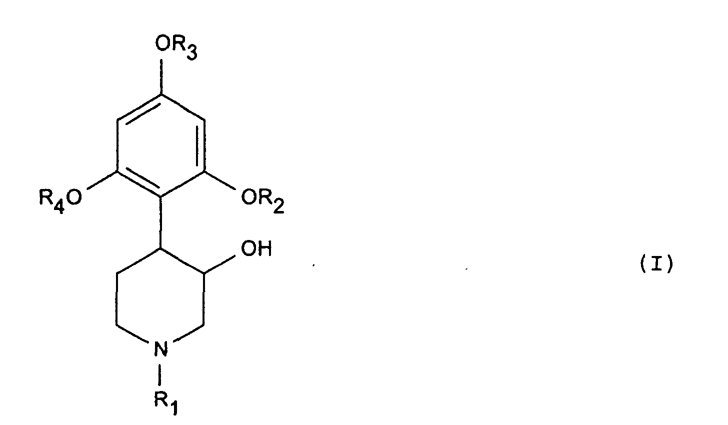 dans laquelle R₁ représente un atome d'hydrogène ou un radical alkyle renfermant jusqu'à 8 atomes de carbone, R₂ et R₄ représentent un radical alkyle renfermant jusqu'à 4 atomes de carbone et R₃ représente un radical alkyle renfermant jusqu'à 8 atomes de carbone, **caractérisé en ce que** l'on soumet un composé de formule (II) : 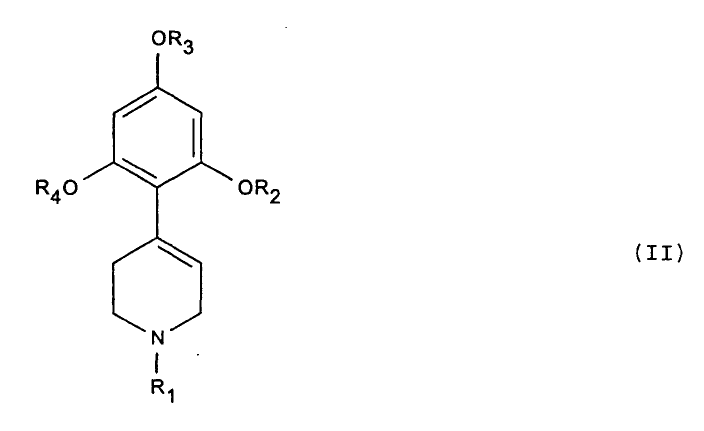 dans laquelle R₁, R₂, R₃ et R₄ conservent leur signification précédente ou un sel d'amine de ce composé, à l'action d'un agent d'halogénation électrophile pour obtenir le composé de formule (III) : 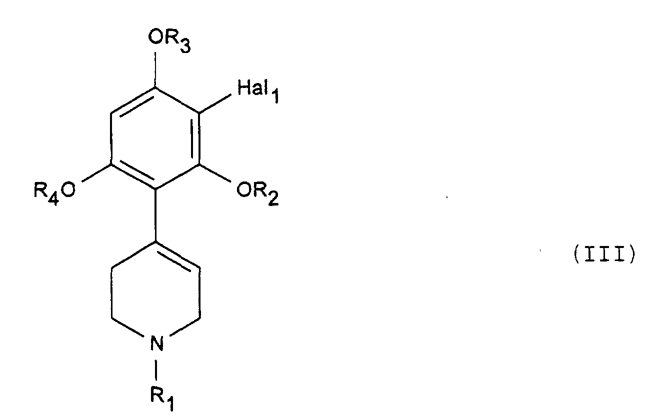 dans laquelle Hal₁ représente un atome d'halogène que l'on soumet à l'action d'un agent d'halohydroxylation pour obtenir le composé de formule (IV) : 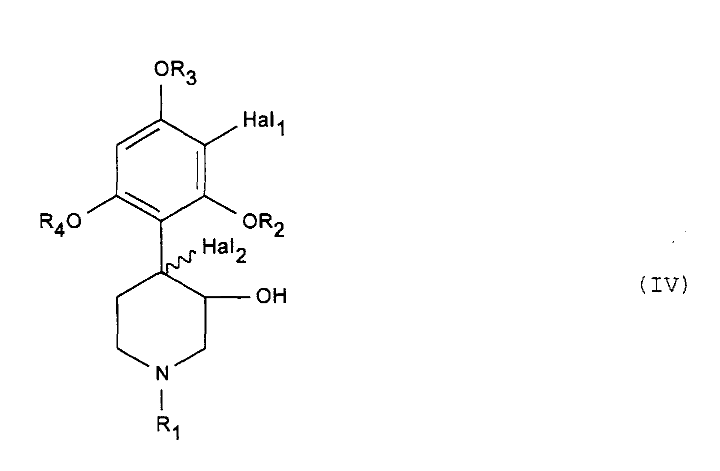 dans laquelle Hal₂ représente un atome d'halogène, que l'on soumet à l'action d'une base, pour obtenir le composé de formule (V) : 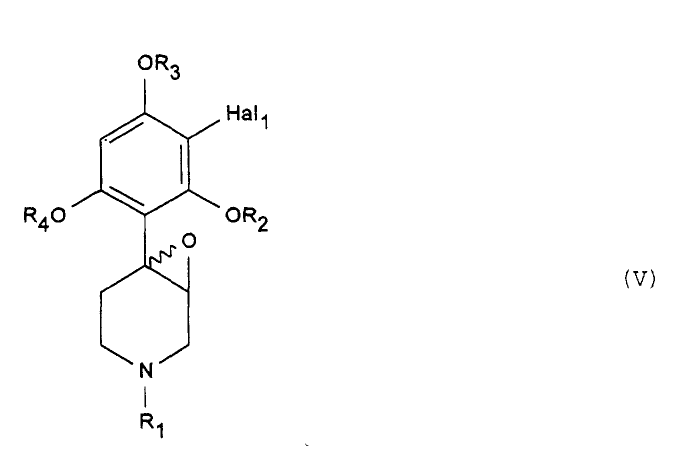 que l'on soumet à l'action d'un agent de réduction diastéréosélective du groupe époxy pour obtenir le composé de formule (VI) : 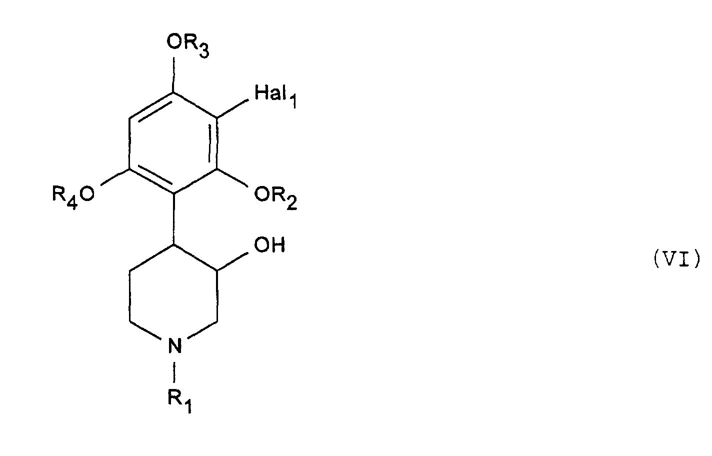 que l'on soumet à l'action d'un agent de réduction pour obtenir le composé racémique cis de formule (I) : 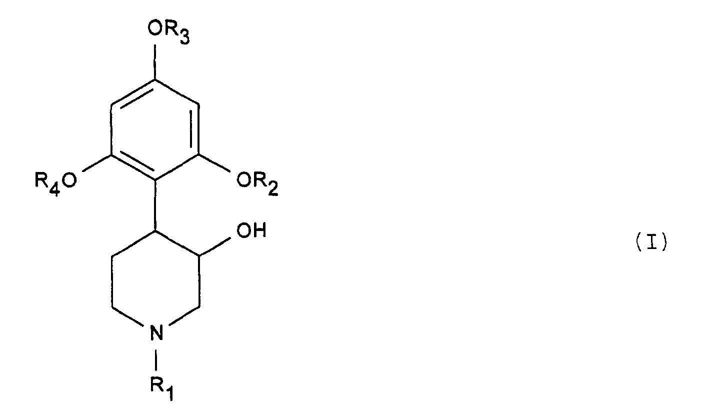 que l'on soumet si désiré, à l'action d'un agent de dédoublement pour obtenir le composé de formule (I) correspondant sous la forme optique voulue.

2. Procédé selon la revendication 1, **caractérisé en ce que** R₁ représente un radical alkyle renfermant jusqu'à 4 atomes de carbone.

3. Procédé selon la revendication 1 ou 2, **caractérisé en ce que** R₂, R₃ et R₄ représentent un radical alkyle renfermant jusqu'à 4 atomes de carbone.

4. Procédé selon la revendication 1, **caractérisé en ce que** R₁, R₂, R₃ et R₄ représentent un radical méthyle.

5. Procédé selon l'une quelconque des revendications 1 à 4, **caractérisé en ce que** le produit de départ est un produit de formule (II) utilisé sous forme de sel d'amine.

6. Procédé selon l'une quelconque des revendications 1 à 5, **caractérisé en ce que** Hal₁ et Hal₂ sont des atomes de brome.

7. Procédé selon la revendication 6, **caractérisé en ce que** l'agent d'halohydroxylation est le N-bromo succinimide.

8. Procédé selon la revendication 7, **caractérisé en ce que** la base utilisée pour créer l'époxy est la soude ou la potasse.

9. Procédé selon l'une quelconque des revendications 1 à 8, **caractérisé en ce que** les composés de formule (III) et (IV) ne sont pas isolés.

10. Procédé selon l'une quelconque des revendications 1 à 9, **caractérisé en ce que** l'agent de réduction diastéréosélective de l'époxy est l'alane AlH₃.

11. Procédé selon l'une quelconque des revendications 1 à 10, **caractérisé en ce que** l'agent de réduction du composé (VI) est le zinc en milieu alcoolique.

12. Procédé selon l'une quelconque des revendications 1 à 11, **caractérisé en ce que** l'agent de dédoublement est un acide de formule : 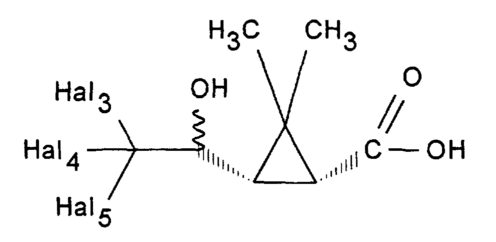 dans laquelle Hal₃ représente un atome de chlore, de brome ou un groupement CF₃ et Hal₄ et Hal₅ représentent un atome de chlore ou de brome.

13. Procédé selon la revendication 12, **caractérisé en ce que** le dédoublement est réalisé au moyen de l'un des 3 acides suivants :

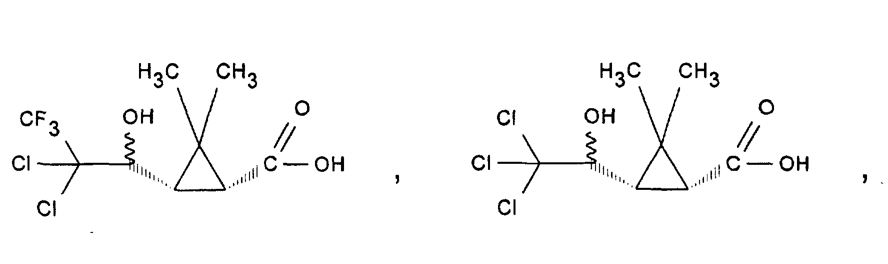

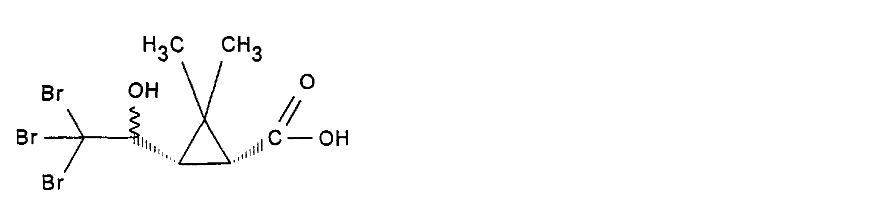

14. Procédé selon la revendication 14, **caractérisé en ce que** l'agent de dédoublement est le :
[1R-(1R*,3S*)] acide 3-(2,2-dichloro-1-hydroxy-3,3,3-trifluoropropyl)-2,2-diméthyl-cyclopropanecarboxylique.

15. Procédé selon l'une quelconque des revendications 1 à 14, **caractérisé en ce que** le produit de départ utilisé est le :
1-méthyl-1,2,3,6-tétrahydro-4-(2,4,6-triméthoxyphényl)-pyridine
et le produit final préparé :
cis(+-)-1-méthyl-4-(2,4,6-triméthoxyphényl)-3-pipéridinol
que l'on dédouble éventuellement pour obtenir le :
[3S-cis(-)]-1-méthyl-4-(2,4,6-triméthoxyphényl)-3-pipéridinol.

16. A titre de produits chimiques les composés de formules (III), (IV), (V) et (VI) définis à la revendication 1.

17. A titre de produits chimiques définis à la revendication 16, les composés suivants :
- 4-(3-bromo-2,4,6-triméthoxyphényl)-1-méthyl-1,2,3,6-tétrahydro-pyridine,
- 4-bromo-4-(3-bromo-2,4,6-triméthoxyphényl)-1-méthyl-3-pipéridinol,
- cis(+-)-4-(3-bromo-2,4,6-triméthoxyphényl)-1-méthyl-3-pipéridinol.

18. A titre de produit chimique défini à la revendication 16, le 6-(3-bromo-2,4,6-triméthoxyphényl)-3-méthyl-7-oxa-3-aza-bicyclo(4,1,0)heptane.

## Claims

1. Method for preparing cis compounds of formula (I): 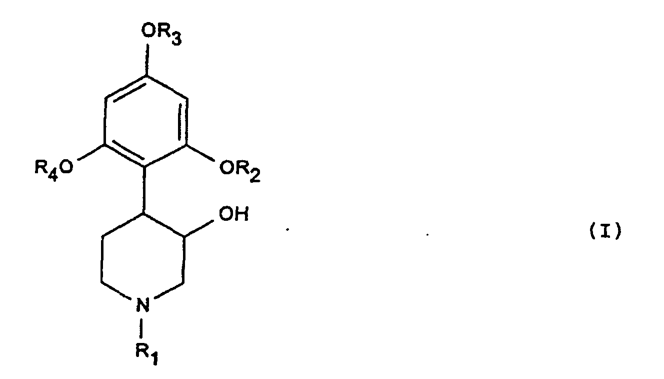 in which R₁ represents a hydrogen atom or an alkyl radical containing up to 8 carbon atoms, R₂ and R₄ represent an alkyl radical containing up to 4 carbon atoms and R₃ represents an alkyl radical containing up to 8 carbon atoms, **characterized in that** a compound of formula (II): 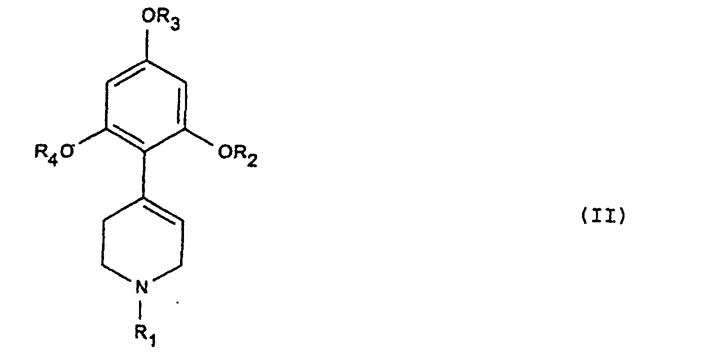 in which R₁, R₂, R₃ and R₄ retain their previous meaning or an amine salt of this compound, is subjected to the action of an electrophilic halogenating agent in order to obtain the compound of the formula (III): 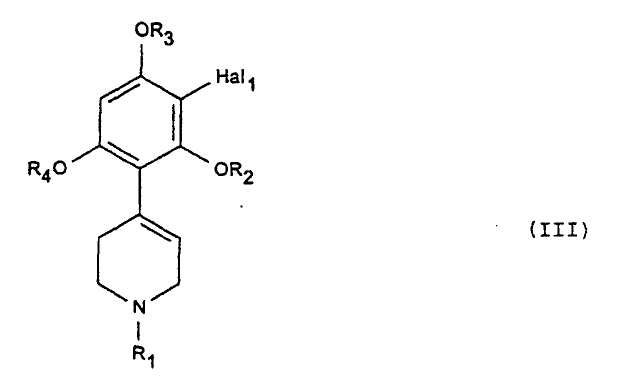 in which Hal₁ represents a halogen atom which is subjected to the action of a halohydroxylating agent in order to obtain the compound of formula (IV): 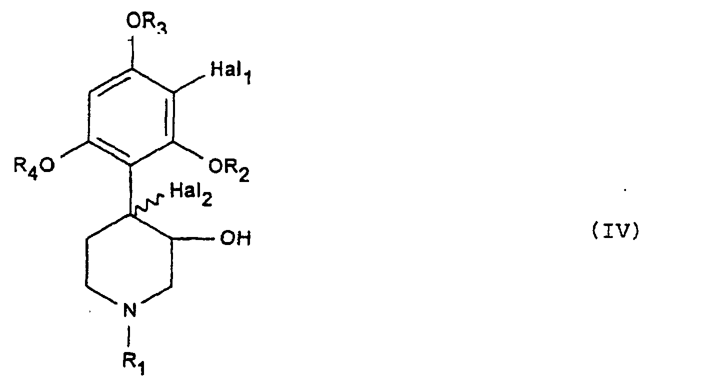 in which Hal₂ represents a halogen atom, which is subjected to the action of a base in order to obtain the compound of formula (V): 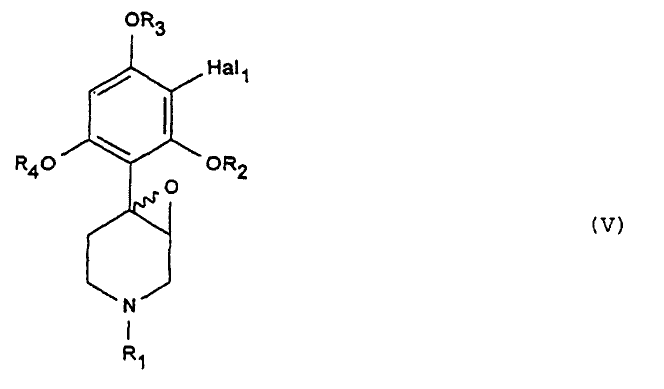 which is subjected to the action of an agent for the diastereoselective reduction of the epoxy group in order to obtain the compound of formula (VI): 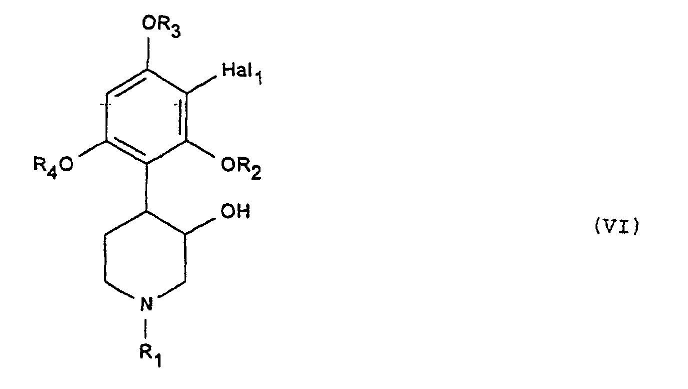 which is subjected to the action of a reducing agent in order to obtain the racemic cis compound of formula (I) : 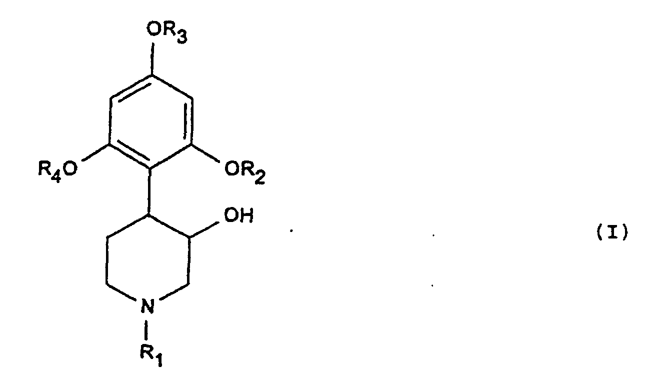 which is subjected, if desired, to the action of a resolving agent in order to obtain the corresponding compound of formula (I) in the desired optical form.

2. Method according to Claim 1, **characterized in that** R₁ represents an alkyl radical containing up to 4 carbon atoms.

3. Method according to Claim 1 or 2, **characterized in that** R₂, R₃ and R₄ represent an alkyl radical containing up to 4 carbon atoms.

4. Method according to Claim 1, **characterized in that** R₁, R₂, R₃ and R₄ represent a methyl radical.

5. Method according to any one of Claims 1 to 4, **characterized in that** the starting material is a product of formula (II) used in the form of an amine salt.

6. Method according to any one of Claims 1 to 5, **characterized in that** Hal₁ and Hal₂ are bromine atoms.

7. Method according to Claim 6, **characterized in that** the halohydroxylating agent is N-bromosuccinimide.

8. Method according to Claim 7, **characterized in that** the base used to create the epoxy is sodium hydroxide or potassium hydroxide.

9. Method according to any one of Claims 1 to 8, **characterized in that** the compounds of formula (III) and (IV) are not isolated.

10. Method according to any one of Claims 1 to 9, **characterized in that** the agent for the diastereoselective reduction of the epoxy is the alane AlH₃.

11. Method according to any one of Claims 1 to 10, **characterized in that** the agent for reducing the compound (VI) is zinc in an alcoholic medium.

12. Method according to any one of Claims 1 to 11, **characterized in that** the resolving agent is an acid of formula: 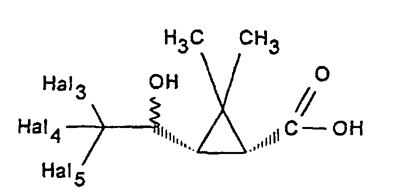 in which Hal₃ represents a chlorine or bromine atom, or a CF₃ group, and Hal₄ and Hal₅ represent a chlorine or bromine atom.

13. Method according to Claim 12, **characterized in that** the resolution is carried out by means of one of the following 3 acids:

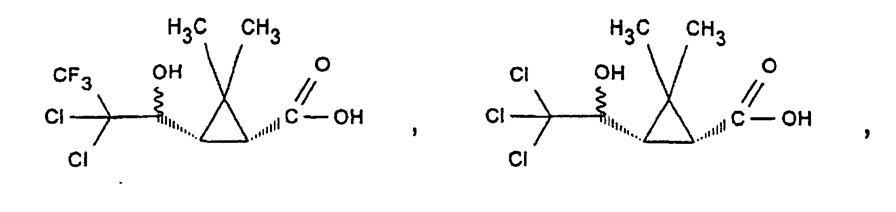

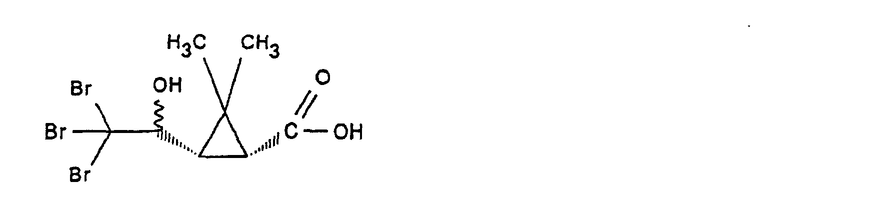

14. Method according to Claim 14, **characterized in that** the resolving agent is:
[1R-(1R*,3S*)]-3-(2,2-dichloro-1-hydroxy-3,3,3-trifluoropropyl)-2,2-dimethylcyclopropanecarboxylic acid.

15. Method according to any one of Claims 1 to 14, **characterized in that** the starting material used is:
1-methyl-1,2,3,6-tetrahydro-4-(2,4,6-trimethoxyphenyl)-pyridine
and the final product prepared:
cis(+-)-1-methyl-4-(2,4,6-trimethoxyphenyl)-3-piperidinol
which is optionally resolved in order to obtain:
[3S-cis(-)]-1-methyl-4-(2,4,6-trimethoxyphenyl)-3-piperidinol.

16. As chemical products, the compounds of formula (III), (IV), (V) and (VI) defined in Claim 1.

17. As chemical products defined in Claim 16, the following compounds:
- 4-(3-bromo-2,4,6-trimethoxyphenyl)-1-methyl-1,2,3,6-tetrahydropyridine,
- 4-bromo-4-(3-bromo-2,4,6-trimethoxyphenyl)-1-methyl-3-piperidinol,
- cis(+-)-4-(3-bromo-2,4,6-trimethoxyphenyl)-1-methyl-3-piperidinol.

18. As a chemical product defined in Claim 16, 6-(3-bromo-2,4,6-trimethoxyphenyl)-3-methyl-7-oxa-3-azabicyclo(4,1,0)heptane.

## Patentansprüche

1. Verfahren zur Herstellung von cis-Verbindungen der Formel (I) 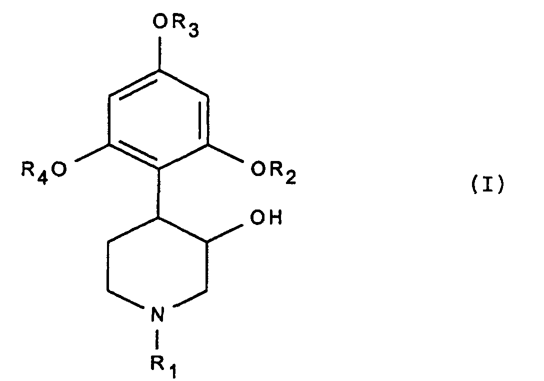 in der R₁ ein Wasserstoffatom oder einen Rest Alkyl mit bis zu 8 Kohlenstoffatomen darstellt, R₂ und R₄ einen Rest Alkyl mit bis zu 4 Kohlenstoffatomen bedeuten und R₃ einen Rest Alkyl mit bis zu 8 Kohlenstoffatomen darstellt, **dadurch gekennzeichnet, daß** man eine Verbindung der Formel (II) 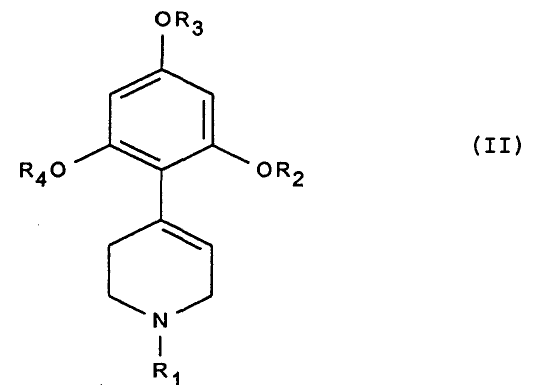 in der R₁, R₂, R₃ und R₄ ihre vorstehende Bedeutung beibehalten, oder ein Aminsalz dieser Verbindung der Einwirkung eines elektrophilen Halogenierungsmittels unterzieht, um die Verbindung der Formel (III) 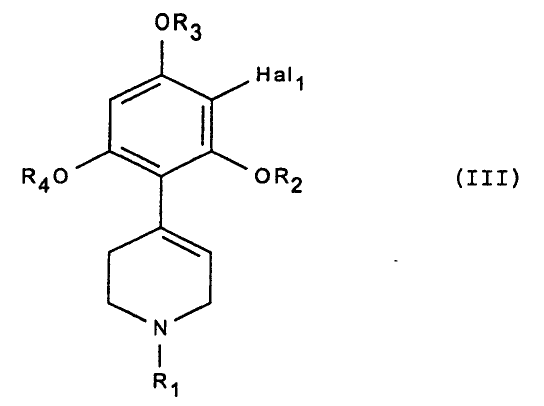 zu erhalten, in der Hal₁ ein Halogenatom darstellt, die man der Einwirkung eines Mittels zur Halohydroxylierung unterzieht, um die Verbindung der Formel (IV) 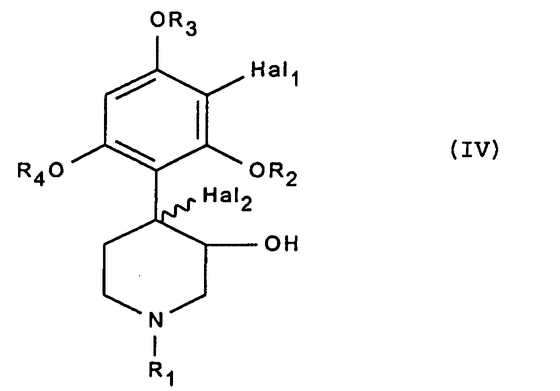 zu erhalten, in der Hal₂ ein Halogenatom darstellt, die man dann der Einwirkung einer Base unterzieht, um die Verbindung der Formel (V) 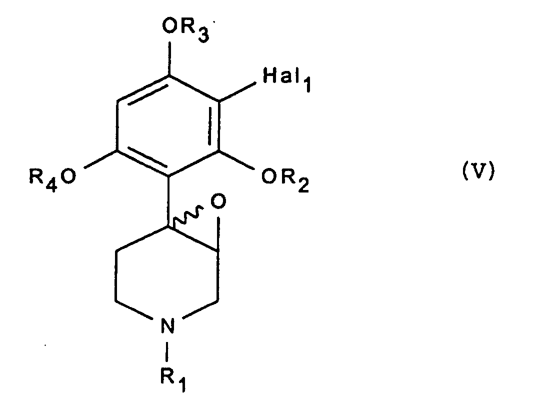 zu erhalten, die man danach der Einwirkung eines Mittels zur diastereoselektiven Reduktion der Gruppe Epoxy unterzieht, um die Verbindung der Formel (VI) 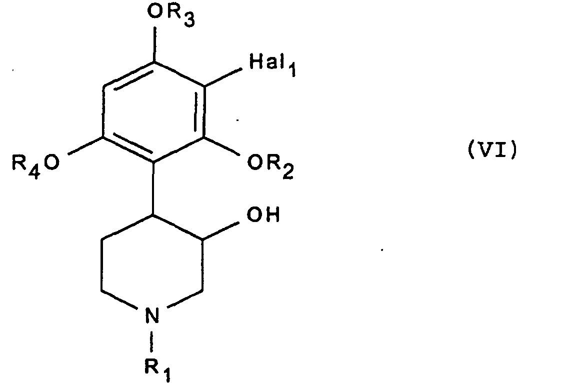 zu erhalten, die man der Einwirkung eines Reduktionsmittels unterzieht, um die racemische cis-Verbindung der Formel (I) 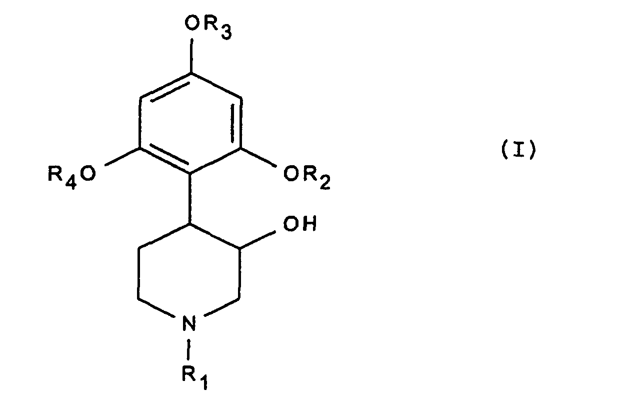 zu erhalten, die man anschließend, wenn erwünscht, der Einwirkung eines Mittels zur Aufspaltung unterzieht, um die entsprechende Verbindung der Formel (I) in der gewünschten optischen Form zu erhalten.

2. Verfahren nach Anspruch 1, **dadurch gekennzeichnet, daß** R₁ einen Rest Alkyl mit bis zu 4 Kohlenstoffatomen darstellt.

3. Verfahren nach Anspruch 1 oder 2, **dadurch gekennzeichnet, daß** R₂, R₃ und R₄ einen Rest Alkyl mit bis zu 4 Kohlenstoffatomen darstellen.

4. Verfahren nach Anspruch 1, **dadurch gekennzeichnet, daß** R₁, R₂, R₃ und R₄ einen Rest Methyl darstellen.

5. Verfahren nach irgendeinem der Ansprüche 1 bis 4, **dadurch gekennzeichnet, daß** das Ausgangsprodukt ein Produkt der Formel (II) ist, das in Form des Aminsalzes verwendet wird.

6. Verfahren nach irgendeinem der Ansprüche 1 bis 5, **dadurch gekennzeichnet, daß** Hall und Hal₂ Bromatome sind.

7. Verfahren nach Anspruch 6, **dadurch gekennzeichnet, daß** das Mittel zur Halohydroxylierung N-Bromsuccinimid ist.

8. Verfahren nach Anspruch 7, **dadurch gekennzeichnet, daß** die Base, die zur Erzeugung des Epoxy verwendet wird, Natriumhydroxid oder Kaliumhydroxid ist.

9. Verfahren nach irgendeinem der Ansprüche 1 bis 8, **dadurch gekennzeichnet, daß** die Verbindungen der Formeln (III) und (IV) nicht isoliert werden.

10. Verfahren nach irgendeinem der Ansprüche 1 bis 9, **dadurch gekennzeichnet, daß** das Mittel zur diastereoselektiven Reduktion des Epoxy Alan AlH₃ ist.

11. Verfahren nach irgendeinem der Ansprüche 1 bis 10, **dadurch gekennzeichnet, daß** das Mittel zur Reduktion der Verbindung (VI) Zink im alkoholischen Medium ist.

12. Verfahren nach irgendeinem der Ansprüche 1 bis 11, **dadurch gekennzeichnet, daß** das Mittel zur Aufspaltung eine Säure der Formel 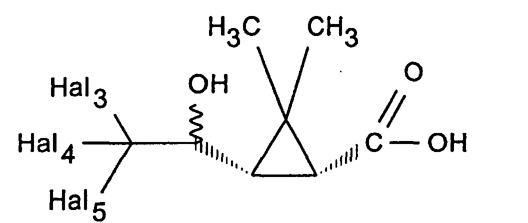 ist, in der Hal₃ ein Atom von Chlor, Brom oder eine Gruppe CF₃ darstellt und Hal₄ und Hal₅ ein Atom von Chlor oder Brom bedeuten.

13. Verfahren nach Anspruch 12, **dadurch gekennzeichnet, daß** die Aufspaltung mit Hilfe von einer der folgenden drei Säuren durchgeführt wird:

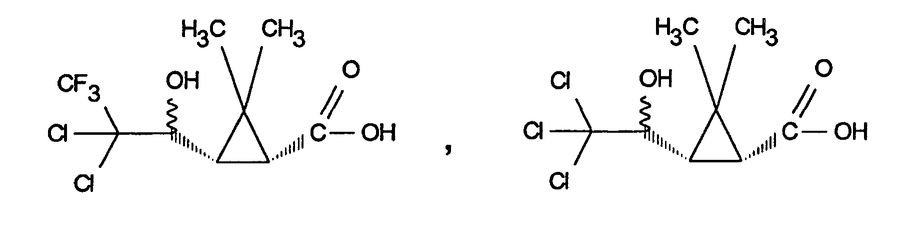

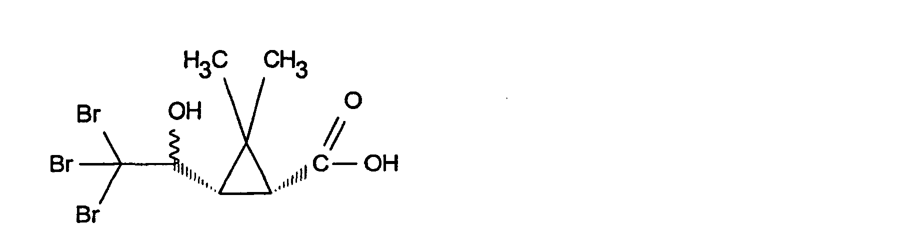

14. Verfahren nach Anspruch 14, **dadurch gekennzeichnet, daß** das Mittel zur Aufspaltung ist:
[1R-(1R*,3S*)]-3-(2,2-Dichlor-1-hydroxy-3,3,3-trifluorpropyl)-2,2-dimethyl-cyclopropancarbonsäure.

15. Verfahren nach irgendeinem der Ansprüche 1 bis 14, **dadurch gekennzeichnet, daß** das verwendete Ausgangsprodukt ist:
1-Methyl-1,2,3,6-tetrahydro-4-(2,4,6-trimethoxyphenyl)-pyridin
und das hergestellt Endprodukt ist:
cis(+-)-1-Methyl-4-(2,4,6-trimethoxyphenyl)-3-piperidinol,
das man gegebenenfalls aufspaltet, um zu erhalten:
[3S-cis(-)]-1-Methyl-4-(2,4,6-trimethoxyphenyl)-3-piperidinol.

16. Als chemische Produkte die Verbindungen der Formeln (III), (IV), (V) und (VI) wie in Anspruch 1 definiert.

17. Als chemische Produkte wie in Anspruch 16 definiert die folgenden Verbindungen:
- 4-(3-Brom-2,4,6-trimethoxyphenyl)-1-methyl-1,2,3,6-tetrahydropyridin,
- 4-Brom-4-(3-brom-2,4,6-trimethoxyphenyl)-1-methyl-3-piperidinol,
- cis(+-)-4-(3-Brom-2,4,6-trimethoxyphenyl)-1-methyl-3-piperidinol.

18. Als chemisches Produkt wie in Anspruch 16 definiert:
6-(3-Brom-2,4,6-trimethoxyphenyl)-3-methyl-7-oxa-3-azabicyclo[4,1,0]-heptan.
